(19) Europäisches Patentamt
European Patent Office
Office européen des brevets

(11) EP 0 538 248 B1

(12) EUROPEAN PATENT SPECIFICATION

(45) Date of publication and mention of the grant of the patent: **11.02.1998 Bulletin 1998/07**

(21) Application number: **90910230.3**

(22) Date of filing: **27.06.1990**

(51) Int Cl.[6]: **C07D 487/04**, A61K 31/47 // (C07D487/04, 209:00, 209:00)

(86) International application number: **PCT/US90/03552**

(87) International publication number: **WO 92/00072 (09.01.1992 Gazette 1992/02)**

# (54) TETRAHYDROISOQUINOLINYLCARBAMATES OF 1,2,3,3A,8,8A-HEXAHYDRO-1,3A,8-TRIMETHYLPYRROLO [2,3-B]INDOLE

TETRAHYDROISOCHINOLINYLCARBAMATE VON 1,2,3,3A,8,8A-HEXAHYDRO-1,3A,8-TRIMETHYLPYRROLO [2,3-B] INDOL

TETRAHYDROISOQUINOLINYLCARBAMATES DE 1,2,3,3A,8,8A-HEXAHYDRO-1,3A,8-TRIMETHYLPYRROLO [2,3-B] INDOLE

(84) Designated Contracting States:
**AT BE CH DE DK ES FR GB IT LI LU NL SE**

(43) Date of publication of application:
**28.04.1993 Bulletin 1993/17**

(73) Proprietor: **HOECHST MARION ROUSSEL, INC.
Cincinnati, Ohio 45215 (US)**

(72) Inventors:
- **HAMER, Russell, Richard, Lee
Far Hills, NJ 07931 (US)**
- **HELSLEY, Grover, Cleveland
Pluckemin, NJ 07978 (US)**
- **GLAMKOWSKI, Edward, Joseph
Warren, NJ 07060 (US)**
- **CHIANG, Yulin
Convent Station, NJ 07961 (US)**
- **FREED, Brian, Scott
Somerset, NJ 08873 (US)**
- **KURYS, Barbara, Elaine
Elmwood Park, NJ 07407 (US)**

(74) Representative: **VOSSIUS & PARTNER
Postfach 86 07 67
81634 München (DE)**

(56) References cited:
**US-A- 4 857 648**

- **CHEMICAL ABSTRACTS, vol. 108, no. 25, 20 June 1988, Columbus, Ohio, US; abstract no. 221937m, & EPA 253 372 (20 January 1988) page 598;**
- **PHARMAZEUTISCHE CHEMIE III-ARZNEISTOFFE, pages 391-395, (1988)**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 538 248 B1

## Description

The present invention relates to tetrahydroisoquinolinylcarbamates of 1,2,3,3a,8,8a-hexahydro-1,3a,8-trimethylpyrrolo[2,3-b]indole of formula (I),

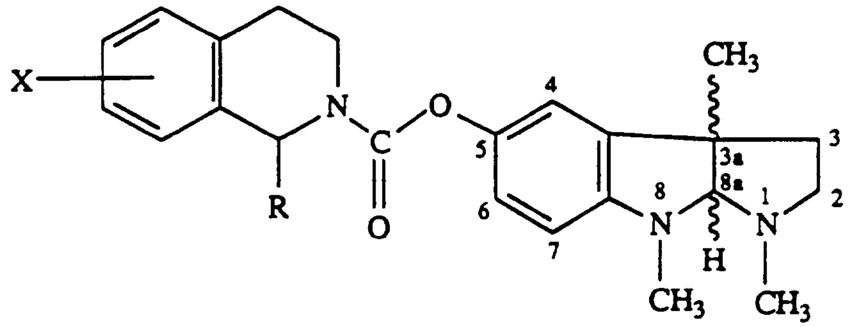

(I)

where R is hydrogen or loweralkyl; and X is hydrogen, loweralkyl, halogen, loweralkoxy or hydroxy, which are useful for alleviating memory dysfunctions characterized by a cholinergic deficit such as Alzheimer's disease.

Unless otherwise stated or indicated, the following definitions shall apply throughout the specification and the appended claims.

The term loweralkyl shall mean a straight or branched alkyl group having from 1 to 6 carbon atoms. Examples of said loweralkyl include methyl, ethyl, n-propyl, isopropyl, n-butyl, iso-butyl, sec-butyl, t-butyl and straight- and branched-chain pentyl and hexyl.

The term halogen shall mean fluorine, chlorine, bromine or iodine.

The compounds of this invention are prepared by utilizing the synthetic steps described below. Throughout the description of the synthetic steps, the notations X and R shall have the respective meanings given above unless otherwise stated or indicated.

In structural formulas depicting the compounds of this invention, heavy lines ( ▬ ) coming out of the 3a-carbon and 8a-carbon of the 1,2,3,3a,8,8a-hexahydropyrrolo[2,3-b]indole ring system signify that the two substituents are above the average plane of the three-ring system, whereas dotted lines ( ┄┄ ) signify that the two substituents are below the average plane of the three-ring system, and wavy lines ( ∿∿ ) signify that the two substituents are both above said average plane or below said average plane. Because of conformational constraints, the two substituents at the 3a- and 8a-positions must be both above said average plane or both below said average plane. Thus, in formula (I), the substituents at the 3a- and 8a-carbons are <u>cis</u> inasmuch as they are on the same side of the three ring system. Where said substituents are both above the average plane of the three ring system, the configuration will be referred to as 3aS-cis and where both substituents are below the average plane of the ring, the configuration will be referred to as 3aR-cis. These two types of configuration are depicted below.

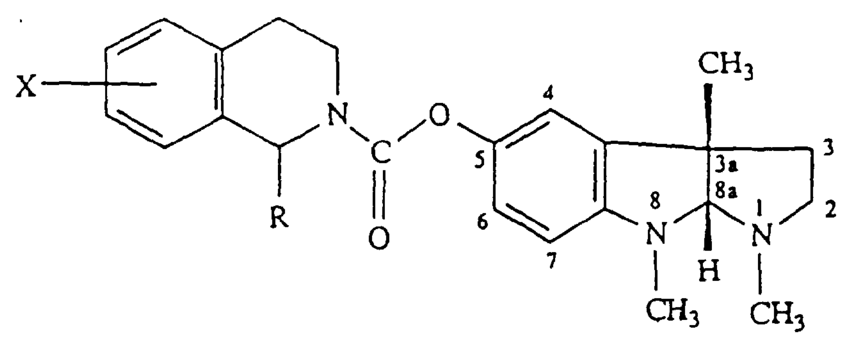

3aS - cis

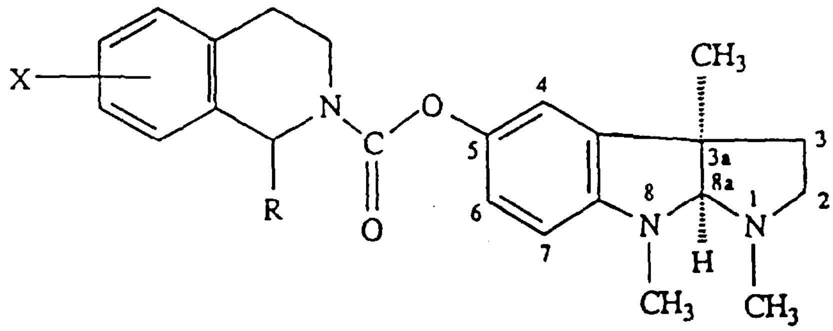

3aR - cis

It is the intent of the present inventors to claim both of said cis isomers, namely, 3aS-cis isomer and 3aR-cis isomer for each given compound name or structural formula containing wavy lines mentioned above. It is also the intent of the present inventors to claim all mixtures of the 3aS-cis and 3aR-cis isomers including the racemic mixture (1:1 ratio of 3aS-cis:3aR-cis).

The compounds of formula I as well as their cis derivatives are not disclosed in the prior art. EP 0 253 372 discloses 1,2,3,3a,8,8a-hexahydro-3a,8 (and 1,3a,8)-di (and -tri) methylpyrrolo/2,3-b/indoles, as well as a process for their preparation and their use as a medicament. However, each of the chemical constituents represents a single ring system containing at least one heteroatom in lieu of the bicyclic ring system disclosed in compound I of the present invention.

**STEP A**

Starting with the compound of formula II and utilizing the synthetic scheme disclosed in Julian et al., J. Chem. Soc., 1935, 563-566 and 755-757, one can prepare the compounds of formulas IV and V. The synthetic scheme is outlined below, but for details the reader is referred to the original articles. For details of another optical resolution procedure not described in Julian et al., the reader is referred to Schonenberger et al., J. Med. Chem., 1986, Volume 29, 2268-2273; and Schonenberger et al., Helv. Chim. Acta, 1986, Volume 69, 283-287 and 1486-1497.

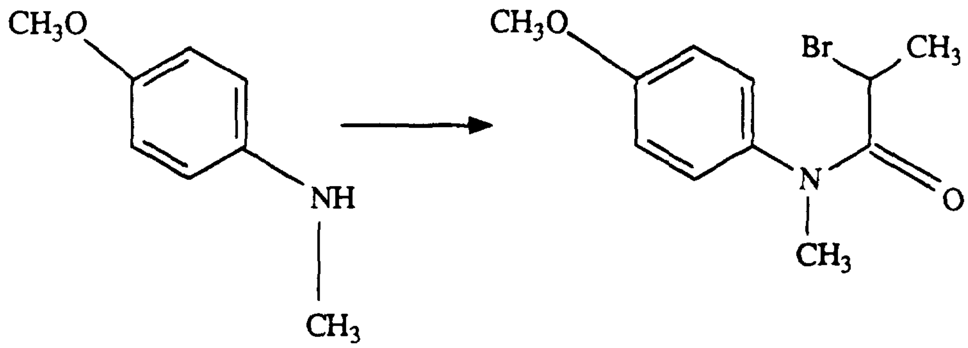

( II )

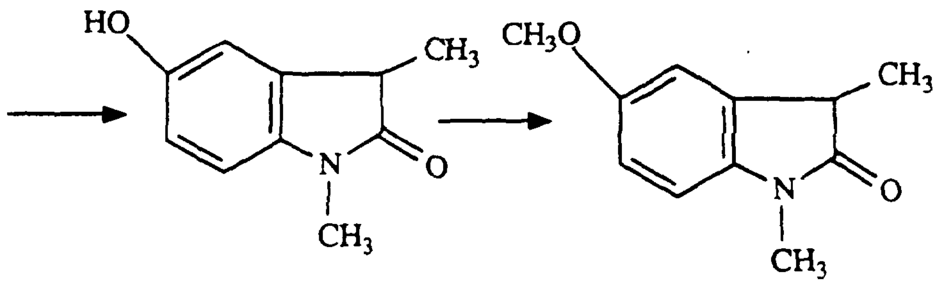

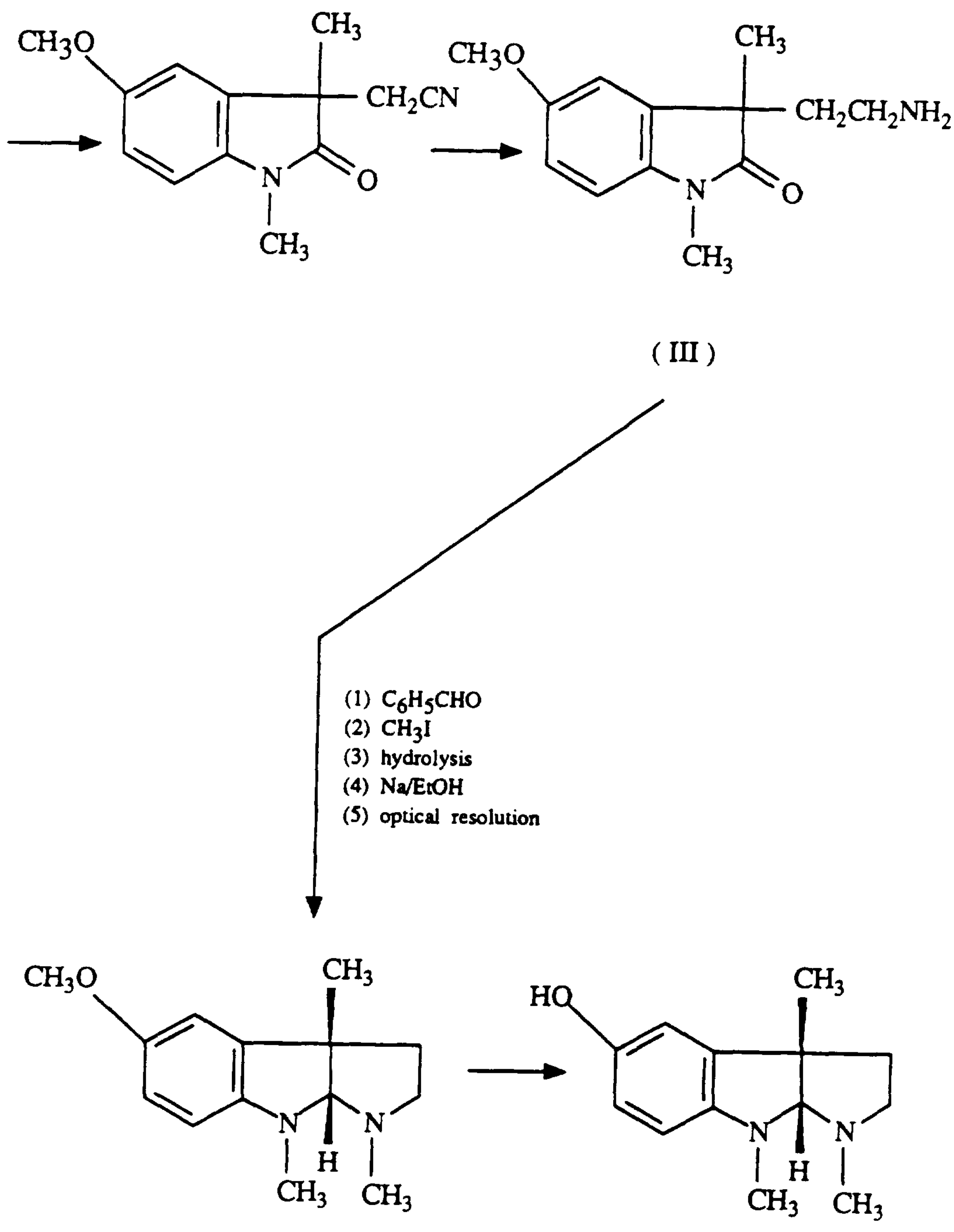


( III )

( IV ) ( V )

If, in the synthetic scheme depicted above, the conversion of compound III to compound IV is conducted without the optical resolution step, a racemic compound is obtained. Said racemic compound is a 50:50 mixture between compound IV and its 3aR-cis isomer, and it can be used for preparing a racemic mixture comprising compound V.

**STEP B**

Compound Va obtained from STEP A is allowed to react with 1,1'-carbonyldiimidazole and the resultant product is allowed to react with an amine of formula VI to afford Compound I.

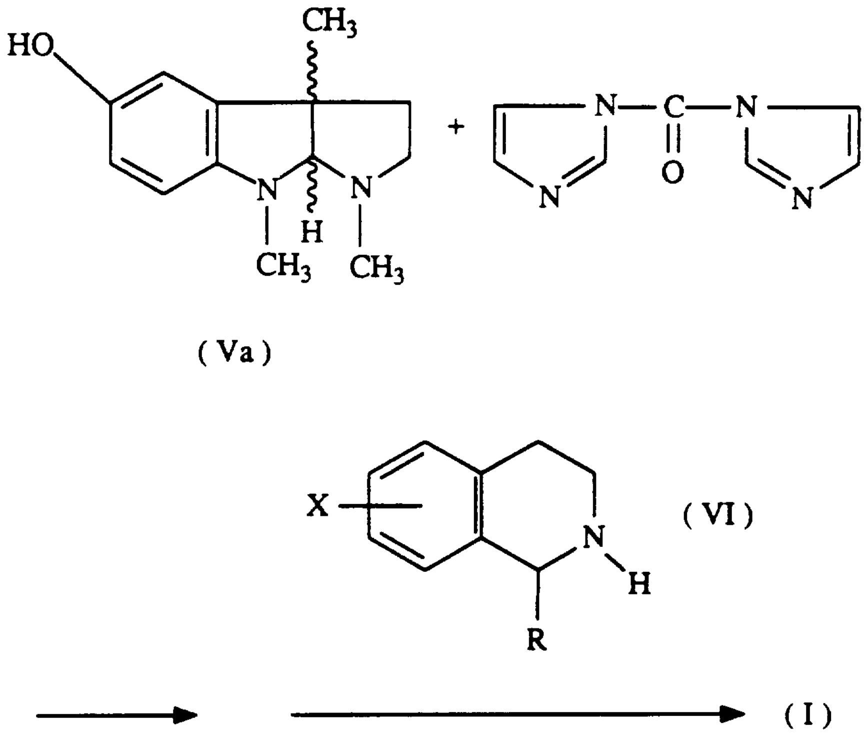

( Va )

( VI )

( I )

Said reaction between compound Va and 1,1'-carbonyldiimidazole is typically conducted by preparing a degassed solution of compound Va in a suitable solvent such as dichloromethane, adding 1,1'-carbonyldiimidazole to the solution and stirring the solution at room temperature for a suitable length of time such as one hour. Said carbamation reaction is typically conducted by adding the amine to the solution obtained above and stirring the solution at room temperature for a few hours.

The compounds of formula I of the present invention are useful for the treatment of various memory dysfunctions characterized by decreased cholinergic function, such as Alzheimer's disease.

This utility is manifested by the ability of these compounds to inhibit the enzyme acetylcholinesterase and thereby increase the acetylcholine levels in the brain.

COMPARISON BETWEEN COMPOUNDS OF THIS INVENTION AND PHYSOSTIGMINE

Although physostigmine is a potent acetylcholinesterase inhibitor, its therapeutic utility is limited by poor stability and oral bioavailability, short duration of action and potent acute toxicity. We have selected (3aS-cis)-1,2,3,3a,8,8a-hexahydro-1,3a,8-trimethylpyrrolo[2,3-b]indol-5-ol,(1,2,3,4-tetrahydroisoquinolinyl)carbamate (Compound A, hereafter) as a representative example of the compounds within the scope of this invention and compared its effects with those of physostigmine is various *in vitro* and *in vivo* tests. The test results have demonstrated that Compound A exhibits several therapeutic advantages over physostigmine.

STABILITY

Compound A was remarkably more stable than physostigmine when incubated with human plasma at 37°C. After 4 hours, approximately 70% of Compound A remained, while physostigmine was completely destroyed under these conditions. Compound A was approximately 34 times less potent as an inhibitor of butyrylcholinesterase than physostigmine. The decreased affinity for this enzyme may contribute to the plasma stability of Compound A.

ORAL BIOAVAILABILITY

Bioavailability studies with Compound A revealed that it was rapidly absorbed orally (Cmax at 30 minutes) and partioned well across the blood brain barrier (brain: plasma at Cmax was 4.55:1). In contrast, physostigmine was so poorly bioavailable by the oral route that pharmacokinetic data was not available. The brain: plasma ratio of physostigmine after intravenous dosing was only approximately 1.5:1 (Somani and Khalique, Drug Metabolism and Disposition,

Volumne 15, 627-633, 1987). The oral bioavailability and activity were also demonstrated in the *ex vivo* acetylcholinesterase (AChE) inhibition tests, in which significant inhibition of rat brain AChE was demonstrated after oral administration of Compound A. (See below for details of the test method used for determining inhibition of rat brain AChE).

DURATION OF ACTION

The duration of action of Compound A was compared with that of physostigmine in the *ex vivo* AChE test. Compound A (20 mg/kg) significantly inhibitied rat brain AChE at 4 and 6 hours after oral administration, while the effects at 24 hours were not statistically significant. A time course of physostigmine action revealed no significant inhibition 2 hours after intraperitoneal adminstration. Repeated administration of 20 mg/kg of Compound A for four days in rats showed no accumulation effect.

POTENCY vs. ACUTE LETHALITY

Compound A and physostigmine are equally potent as inhibitors of AChE *in vitro.* The $IC_{50}$ value for Compound A in this assay was 0.036 $\mu$M and the $IC_{50}$ value for physostigmine was 0.034 $\mu$M. However, there is a substantial difference in acute toxicity between these two compounds. Intraperitonial administration of physostigmine to rats killed 50% of the test animals at dosages between 1.0 and 2.5 mg/kg, while the dose of Compound A that caused 50% lethality was between 40 and 80 mg/kg. There were also substantial differences in cardiovascular effects between Compound A and physostigmine which were apparently due to the noradrenergic effects of Compound A. Compound A (100 $\mu$M) stimulated [$^3$H]norepinephrine release *in vitro,* whereas physostigmine had no such effect. (See below for details of the test method used for determining [$^3$H]norepinephrine release).

Cholinesterase Inhibition Assay

Cholinesterases are found throughout the body, both in the brain and in serum. However, only brain acetylcholinesterase (AChE) distribution is correlated with central cholinergic innervation. This same innervation is suggested to be weakened in Alzheimer patients. We have determined in vitro inhibition of acetylcholinesterase activity in rat striatum.

In Vitro Inhibition of Acetylcholinesterase Activity in Rat Striatum

Acetylcholinesterase (AChE), which is sometimes called true or specific cholinesterase, is found in nerve cells, skeletal muscle, smooth muscle, various glands and red blood cells. AChE may be distinguished from other cholinesterases by substrate and inhibitor specificities and by regional distribution. Its distribution in brain roughly correlates with cholinergic innervation and subfractionation shows the highest level in nerve terminals.

It is generally accepted that the physiological role of AChE is the rapid hydrolysis and inactivation of acetylcholine. Inhibitors of AChE show marked cholinominetic effects in cholinergically-innervated effector organs and have been used thereapeutically in the treatment of glaucoma, myasthenia gravis and paralytic ileus. However, recent studies have suggested that AChE inhibitors may also be beneficial in the treatment of Alzheimer's dementia.

The method described below was used in this invention for assaying cholinesterase activity. This is a modification of the method of Ellman et al., Biochem. Pharmacol. 7, 98 (1961).

Procedure:

A. Reagents -

1. 0.05 M Phosphate buffer, pH 7.2

(a) 6.85 g $NaH_2PO_4 \cdot H_2O$/100 ml distilled $H_2O$
(b) 13.40 g $Na_2HPO_4 \cdot 7H_2O$/100 ml distilled $H_2O$
(c) add (a) to (b) until pH reaches 7.2
(d) Dilute 1:10

2. Substrate in buffer

(a) 198 mg acetylthiocholine chloride (10 mM)
(b) q.s to 100 ml with 0.05 M phosphate buffer, pH 7.2 (reagent 1)

3. DTNB in buffer

(a) 19.8 mg 5,5-dithiobisnitrobenzoic acid (DTNB) (0.5 mM)
(b) q.s. to 100 ml with 0.05 M phosphate buffer, pH 7.2 (reagent 1)

4. A 2 mM stock solution of the test drug is made up in a suitable solvent and q.s. to volume with 0.5 mM DTNB (reagent 3). Drugs are serially diluted (1:10) such that the final concentration (in cuvette) is $10^{-4}$ M and screened for activity. If active, $IC_{50}$ values are determined from the inhibitory activity of subsequent concentrations.

B. Tissue Preparation-
Male Wistar rats are decapitated, brains rapidly removed, copora striata disected free, weighed and homogenized in 19 volumes (approximately 7 mg protein/ml) of 0.05 M phosphate buffer, pH 7.2 using a Potter-Elvehjem homogenizer. A 25 microliter aliquot of the homogenate is added to 1.0 milliter vehicle or various concentrations of the test drug and preincubated for 10 minutes at 37°C.
C. Assay-
Enzyme activity is measured with the Beckman DU-50 spectrophotometer. This method can be used for $IC_{50}$ determinations and for measuring kinetic constants.
Instruments Settings

Kinetics Soft-Pac Module #598273 (10)
Program #6 Kindata:
Source - Vis
Wavelength - 412 nm
Sipper - none
Cuvettes - 2 ml cuvettes using auto 6-sampler
Blank - 1 for each substrate concentration
Interval time - 15 seconds (15 or 30 seconds for kinetics)
Total time - 5 minutes (5 or 10 minutes for kinetics)
Plot - yes
Span - autoscale
Slope - increasing
Results - yes (gives slope)
Factor - 1

Reagents are added to the blank and sample cuvettes as follows:

| | |
|---|---|
| Blank: | 0.8 ml Phosphate Buffer/DTNB |
| | 0.8 ml Buffer/Substrate |
| Control: | 0.8 ml Phosphate Buffer/DTNB/Enzyme |
| | 0.8 ml Phosphate Buffer/Substrate |
| Drug: | 0.8 ml Phosphate Buffer/DTNB/Drug/ Enzyme |
| | 0.8 ml Phosphate Buffer/Substrate |

Blank values are determined for each run to control for non-enzymatic hydrolysis of substrate and these values are automatically subtracted by the kindata program available on kinetics soft-pac module. This program also calculates the rate of absorbance change for each cuvette.

For $IC_{50}$ Determinations:

Substrate concentration is 10 mM diluted 1:2 in assay yielding final concentration of 5 mM. DTNB concentration is 0.5 mM yielding 0.25 mM final concentration.

$$\% \text{ Inhibition} = \frac{\text{slope control - slope drug}}{\text{slope control}} \times 100$$

$IC_{50}$ values are calculated from log-probit analysis.

$^3$H-Norepinephrine Uptake in Rat Whole Brain or Hypothalamic Synaptosomes

This asay is used as a biochemical screen for compunds which block norepinephrine uptake.

The neuronal re-uptake mechanism for norepinephrine (NE) is the most important physiological means for inactivating NE by removing the transmitter from the synaptic cleft. NE uptake is accomplished by a saturable, sterospecific, high-affinity ($K_m$=$10^{-7}$ -$10^{-6}$ M), sodium-dependent, active transport system, which has been shown to exist in both peripheral and central nervous system tissues, using slice, homogenate and purified synaptosome preparations. NE uptake is potently inhibited by cocaine, phenethylamines and tricyclic antidepressants. It is also inhibited by ouabain, metabolic inhibitors and phenoxybenzamine. The inhibition of NE uptake by clinically effective tricyclic antidepressants is an important link in the catecholamine hypothesis of affective disorders.

There are large regional variations in NE uptake which correlate with the endogenous levels of NE. The hypothalamus shows the highest level of NE and the greatest uptake. This region is used for further testing of compounds showing activity in whole brain preparations.

Synaptosomal $^3$H-NE uptake is a useful marker for the integrity of noradrenergic neurons, after lesioning experiments, as well as an assay for compounds which potentiate the action of NE by blocking the reuptake mechanism.

Procedure:

A. Animals: Male CR Wistar rats (100-125 g)

B. Reagents-

1. Krebs-Henseleit Bicarbonate Buffer, pH 7.4 (KHBB).
Make a 1 liter batch, containing the following salts.

| | grams/L | mM |
|---|---|---|
| NaCl | 6.92 | 118.4 |
| KCL | 0.35 | 4.7 |
| $MgSO_4 \cdot 7H_2O$ | 0.29 | 1.2 |
| $KH_2PO_4$ | 0.16 | 2.2 |
| $NaHCO_3$ | 2.10 | 24.9 |
| $CaCl_2$ | 0.14 | 1.3 |
| Prior to use add: | | |
| Dextrose | 2 mg/ml | 11.1 |
| Iproniazid phosphate | 0.30 mg/ml | 0.1 |

Aerate for 60 min. with 95% $O_2$/5% $CO_2$, check pH (7.4 ± 0.1).

2. 0.32 M Sucrose: 21.9 g of surose, q.s to 200 ml.

3. L(-)-Norepinephrine bitartrate is procured from a commerical source. A 0.1 mM stock solution is made up in 0.01 N HCl. This is used to dilute the specific activity of the radiolabeled NE.

4. Levo-[Ring-2,5,6-$^3$H]-Norepinephrine (40-50 Ci/mmol) is obtained from a commercial source. The final desired concentration of $^3$H-NE in the assay is 50 nM. The dilution factor is 0.8; therefore the KHBB is made up to contain 62.5 nM[$^3$H]-NE.

Add to 100 ml of KHBB:

| | | |
|---|---|---|
| A. | 59.4 microliter of 0.1 mM NE = | 59.4 nM |
| B. | 0.31 nmole of $^3$H-NE = | 3.1 nM |
| | | 62.5 nM |

5. For most assays, a 1 mM stock solution of the test compound is made up in a suitable solvent and serially diluted such that the final concentration in the assay ranges from $2x10^{-8}$ to $2x10^{-5}$ M. Seven concentrations are used for each assay. Higher or lower concentrations may be used depending on the potency of the test compound.

C. Tissue Preparation

Male Wister rats are decapitated and brains rapidly removed. Either whole brain minus cerebella or hypothalamus is weighed and homogenized in 9 volumes of ice-cold 0.32 M sucrose using a Potter-Elvejhem homogenizer. Homogenization should be done with 4-5 up and down strokes at medium speeds to minimize synaptosome lysis. The homogenate is centrifuged at 1000 g for 10 min. at 0-4°C. The supernatant ($S_1$) is decanted and is used for uptake experiments.

D. Assay

| | |
|---|---|
| 800 microliter | KHBB containing [$^3$H]-NE |
| 20 microliter | Vehicle or appropriate drug concentration |
| 200 microliter | Tissue suspension |

Tubes are incubated at 37°C under 95% $O_2$/5% $CO_2$ atmosphere for five minutes. For each asay, 3 tubes are incubated with 20 microliters of vehicle at 0°C in an ice bath. After incubation all tubes are immediately centrifuged at 4000 g for ten minutes. The supernatant fluid is aspirated and the pellets dissolved by adding 1 ml of a solubilizer. The tubes are vigorously vortexed, decanted into scintillation vials, and counted in 10 ml of Liquiscint scintillation counting cocktail. Active uptake is the difference between cpm at 37°C and 0°C. The percent inhibition at each drug concentration is the mean of three determinations. $IC_{50}$ values are derived from log-probit analysis.

CARDIOVASCULAR PHARMACOLOGY

**Objective:** The purpose of the study described below was to characterize the cardiovascular profile of Compound A and to compare it with that of physostigmine, a standard acetylcholinesterase inhibitor. (Details of test methods used for determining acute cardiovascular hemodynamic effects are presented following the Conclusion section).

**Summary of Results:** Compound A infused intravenously (0.01 mg/kg/min) in anesthetized dogs did not elicit the prominent cardiovascular depression as observed for equivalent rates of physostigmine infusions (See TABLE 1). In fact, at higher doses of Compound A (0.1 mg/kg/min, i.v.), the measured cardiac and peripheral vascular parameters were markedly increased (TABLE 1). This hemodynamic profile is qualitatively similar to that observed for intravenous injections of the endogenous catecholamine, norepinephrine. Thus, the cardiovascular responses to Compound A are also consistent with in vitro results showing that Compound A enhances the spontaneous release of norepinephrine from sympathetic nerve terminals.

Supplemental studies in anesthetized dogs were carried out comparing the cardiovascular effects of Compound A (0.1 mg/kg/min i.v.) and physostigmine (0.01 mg/kg/min i.v.) during combined blockade of alpha and beta adrenergic receptors. Physostigmine infusions during alpha and beta receptor blockade resulted in severe cardiovascular depression and mortality within 17-29 minutes in 3 out of 3 dogs. There was no mortality observed at this dose of physostigmine with intact alpha and beta adrenergic receptors. In contrast, alpha and beta adrenergic blockade abolished the positive cardiac and hemodynamic effects of Compound A resulting in moderate decreases in blood pressure and cardiac output.

The higher dose of physostigmine utilized in these studies produced blockade of the conduction of action potentials from the atria to the ventricles (A-V block; TABLE 1). There were no drug related changes in the electrocardiogram (ECG) at 0.01-0.1 mg/kg/min (i.v.) of Compound A. The notable increases in right atrial pressure (RAP) and left ventricular end-diastolic pressure (LVEDP) observed during physostigmine infusions (indicative of a depression in the contractile strength of the heart) were not observed during Compound A infusions.

**Conclusions:** The results summarized above suggest that although Compound A exhibits potent acetylocholinesterase inhibition in dogs, it possesses a unique property in attenuating cardiovascular depression as observed with the standard, physostigmine. In addition, the data show the importance of functional adrenergic compensatory mechanisms in attenuating severe cardiovascular depression caused by acetylcholinesterase inhibition.

METHODS USED FOR ACUTE CARDIOVASCULAR HEMODYNAMIC EVALUATION

Beagles of either sex are anesthetized with sodium thiopental 15 mg/kg + sodium barbital 200 mg/kg + 60 mg of sodium pentobarbital i.v. The trachea is intubated with a cuffed endotracheal tube and connected to a Harvard Respirator Pump set at 20 ml/stroke and 10 strokes/minute. The right femoral artery and vein are exposed and cannulated with polyethylene tubing for measurement of arterial blood pressure and i.v. administration of drugs, respectively. The duration of these experiments is two hours postdrug when drug is administered i.v. and three hours when administered i.d.

The arterial cannula is connected to a Statham P23Gb transducer. Left ventricular cardiac catheterization is accomplished by introducing a Millar microtip pressure transducer into the left carotid artery and advancing it to the left ventricle. The cannula is connected to a Millar TC-100 transducer control unit which is connected to a Beckman voltage/pressure pulse coupler. Ventricular pressure and its first derivative dP/dt are measured simultaneously by wiring the signal output from the left ventricular pressure tracing into a differentiator. Cardiac output (CO) is determined by thermodilution technique using a quadruple lumen 7F Swan-Ganz flow directed catheter introduced into the right external jugular vein and threaded into the superior vena cava, right atrium, through the right ventricle and into the pulmonary artery. The proximal lumen of this catheter is in the right atrium when the distal lumen and thermistor have been properly placed in the pulmonary artery. The signal outputs from the proximal and distal sites are connected to Statham P23BB transducers to measure right atrial pressure (RAP) and pulmonary artery pressure (PAP). Cardiac output is determined by injecting 5 ml of iced 5% dextrose in water into the proximal site (right atrium) and then measuring the temperature change in the pulmonary artery. Using the Stewart-Hamilton equation, the cardiac output can be determined by integrating the area under the thermodilution curve with an Edwards Cardiac Output Computer (model 9520). Heart rate is determined with a Beckman Cardiotach which sums the pressure waves from the pulse pressure curve of the arterial pressure. Lead II EKG is recorded. All of these signal outputs are displayed on a Beckman R-611 recorder. The measurement of these parameters provides information so that the following may be either measured directly or calculated:

1. Mean Arterial Blood Pressure - MAP (mmHg)
2. Heart Rate - HR
3. Cardiac Output - CO (1/minute)
4. Total Peripheral Resistance - TPR
5. Cardiac Index - CI ($CO/m^2$)
6. Stroke Volume - SV (ml/beat)
7. Stroke Work - SW (gm-m/beat)
8. Central Venous Pressure - CVP measured as right atrial pressure (RAP) mmHg or $cmH_2O$.
9. Left Ventricular End Diastolic Pressure - LVEDP (mmHg)
10. Rate of Rise of Ventricular Pressure - dP/dt/Pmax

    This parameter is a better estimate of the contractile state of the myocardium that dP/ dt alone since the former is relatively independent of the loading conditions of the heart ($sec^{-1}$).
11. Plumonary Artery Pressure - PAP (mmHg)
12. Electrocardiogram - EKG

Results of the acute cardiovascular hemodymic evaluation for Compound A and physostigmine are presented in Table 1.

TABLE 1

| COMPARISON OF THE HEMODYNAMIC EFFECTS IN THE ANESTHETIZED DOG | | | | |
|---|---|---|---|---|
| Dose | 0.01 mg/kg/min. | | 0.1 mg/kg/min. | |
| Route | i.v. | | i.v. | |
| | Compound A | Physostigmine | Compound A | Physostigmine* |
| MAP | -12% | -49% | +54% | -78% |
| HR | -12% | -49% | -13% | -82% |
| CO | +20/-17% | -23% | +40% | -79% |
| TRP | -18% | -34% | +61% | -48% |
| dP/dT | +37% | -32% | +38% | -80% |
| RAP | NC | +5mmHG | NC | +8mmHG |
| LVEDP | NC | +5mmHG | NC | +8mmHG |
| ECG | NC | NC | NC | A-V block inc. T wave |

* N=2/3 dogs; N=1 dog expired within 30 min.

Effective quantities of the compounds of the invention may be administered to a patient by any of the various methods, for example, orally as in capsule or tablets, parenterally in the form of sterile solutions or suspensions, and in some cases intravenously in the form of sterile solutions. The free base final products, while effective themselves, may be formulated and administered in the form of their pharmaceutically acceptable acid addition salts for purposes of stability, convenience of crystallization, increased solubility and the like.

Acids useful for preparing the pharmaceutically acceptable acid addition salts of the invention include inorganic acids such as hydrochloric, hydrobromic, sulfuric, nitric, phosphoric and perchloric acids, as well as organic acids such as tartaric, citric, acetic, succinic, maleic, fumaric and oxalic acids.

The active compounds of the present invention may be orally administered, for example, with an inert diluent or with an edible carrier, or they may be enclosed in gelatin capsules, or they may be compressed into tablets. For the purpose of oral therapeutic administration, the active compounds of the invention may be incorporated with excipients and used in the form of tablets, troches, capsules, elixirs, suspensions, syrups, wafers, chewing gum and the like. These preparations should contain at least 0.5% of active compounds, but may be varied depending upon the particular form and may conveniently be between 4% to about 70% of the weight of the unit. The amount of active compound in such compositions is such that a suitable dosage will be obtained. Preferred compositions and preparations according to the present invention are prepared so that an oral dosage unit form contains between 1.0 - 300 milligrams of active compound.

The tablets, pills, capsules, troches and the like may also contain the following ingredients: a binder such as microcrystalline cellulose, gum tragacanth or gelatin; an excipient such as starch or lactose, a disintegrating agent such as alginic acid, Primogel, cornstarch and the like; a lubricant such as magnesium stearate or Sterotex; a glidant such as colloidal silicon dioxide; and a sweeting agent such as sucrose or saccharin may be added or a flavoring agent such as peppermint, methyl salicylate, or orange flavoring. When the dosage unit form is a capsule, it may contain, in addition to materials of the above type, a liquid carrier such as a fatty oil. Other dosage unit forms may contain other various materials which modify the physical form of the dosage unit, for example, as coatings. Thus, tablets or pills may be coated with sugar, shellac, or other enteric coating agents. A syrup may contain, in addition to the active compounds, sucrose as a sweetening agent and certain preservatives, dyes, coloring and flavors. Materials used in preparing these various compositions should be pharmaceutically pure and non-toxic in the amounts used.

For the purpose of parenteral therapeutic administration, the active compounds of the invention may be incorporated into a solution or suspension. These preparations should contain at least 0.1% of active compound, but may be varied between 0.5 and about 30% of the weight thereof. The amount of active compound in such compositions is such that a suitable dosage will be obtained. Preferred compositions and preparations according to the present invention are prepared so that a parenteral dosage unit contains between 0.5 to 100 milligrams of active compound.

The solutions or suspensions may also include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. The parenteral preparation can be enclosed in disposable syringes or multiple dose vials made of glass or plastic.

Examples of the compounds of this invention include those listed below as well as the 3aR-cis isomers thereof and mixtures of the 3aS-cis and 3aR-cis isomers including the racemic mixtures:

(3aS-cis)-1,2,3,3a,8,8a-hexahydro-1,3a,8-trimethylpyrrolo[2,3-b]indol-5-ol, (1,2,3,4-tetrahydroisoquinolinyl)carbamate;

(3aS-cis)-1,2,3,3a,8,8a-hexahydro-1,3a,8-trimethylpyrrolo[2,3-b]indol-5-ol, (1-methyl-1,2,3,4-tetrahydroisoquinolinyl)carbamate;

(3aS-cis)-1,2,3,3a,8,8a-hexahydro-1,3a,8-trimethylpyrrolo[2,3-b]indol-5-ol, (1-ethyl-1,2,3,4-tetrahydroisoquinolinyl)carbamate;

(3aS-cis)-1,2,3,3a,8,8a-hexahydro-1,3a,8-trimethylpyrrolo[2,3-b]indol-5-ol, (1-propyl-1,2,3,4-tetrahydroisoquinolinyl)carbamate;

(3aS-cis)-1,2,3,3a,8,8a-hexahydro-1,3a,8-trimethylpyrrolo[2,3-b]indol-5-ol, (1-butyl-1,2,3,4-tetrahydroisoquinolinyl)carbamate;

(3aS-cis)-1,2,3,3a,8,8a-hexahydro-1,3a,8-trimethylpyrrolo[2,3-b]indol-5-ol, (6-chloro-1,2,3,4-tetrahydroisoquinolinyl)carbamate;

(3aS-cis)-1,2,3,3a,8,8a-hexahydro-1,3a,8-trimethylpyrrolo[2,3-b]indol-5-ol, (7-chloro-1,2,3,4-tetrahydroisoquinolinyl)carbamate;

(3aS-cis)-1,2,3,3a,8,8a-hexahydro-1,3a,8-trimethylpyrrolo[2,3-b]indol-5-ol, (6-chloro-1-methyl-1,2,3,4-tetrahydroisoquinolinyl) carbamate;

(3aS-cis)-1,2,3,3a,8,8a-hexahydro-1,3a,8-trimethylpyrrolo[2,3-b]indol-5-ol, (7-chloro-1-methyl-1,2,3,4-tetrahydroisoquinolinyl) carbamate;

(3aS-cis)-1,2,3,3a,8,8a-hexahydro-1,3a,8-trimethylpyrrolo[2,3-b]indol-5-ol, (6-hydroxy-1,2,3,4-tetrahydroisoquinolinyl) carbamate;

(3aS-cis)-1,2,3,3a,8,8a-hexahydro-1,3a,8-trimethylpyrrolo[2,3-b]indol-5-ol, (7-hydroxy-1,2,3,4-tetrahydroisoquinolinyl) carbamate;

(3aS-cis)-1,2,3,3a,8,8a-hexahydro-1,3a,8-trimethylpyrrolo[2,3-b]indol-5-ol, (6-hydroxy-1-methyl-1,2,3,4-tetrahydroisoquinolinyl) carbamate; and

(3aS-cis)-1,2,3,3a,8,8a-hexahydro-1,3a,8-trimethylpyrrolo[2,3-b]indol-5-ol, (7-hydroxy-1-methyl-1,2,3,4-tetrahydroisoquinolinyl) carbamate.

The following examples are presented in order to illustrate this invention.

**EXAMPLE 1**

(3aS-cis)-1,2,3,3a,8,8a-Hexahydro-1,3a,8-trimethylpyrrolo[2,3-b]indol-5-ol, (1,2,3,4-tetrahydroisoquinolinyl) carbamate

A degassed solution of eseroline (3.0 g) in 80 ml of dry dichloromethane was treated in one portion with 1,1'-carbonyldiimidazole (2.7 g) and stirred at room temperature. After one hour, the solution was treated with 1,2,3,4-tetrahydroisoquinoline (4.0 g) and stirring was continued overnight. The solution was concentrated and the residue purified by flash chromatography to give 2.8 g of pale oil which was crystallized from ether to give 2.4 g of white crystals, mp 83-85°C.

| ANALYSIS: | | | |
|---|---|---|---|
| Calculated for $C_{23}H_{27}N_3O_2$: | 73.18%C | 7.20%H | 11.13%N |
| Found: | 72.98%C | 7.22%H | 11.09%N |

**EXAMPLE 2**

(3aS-cis)-1,2,3,3a,8,8a-Hexahydro-1,3a,8-trimethylpyrrolo[2,3-b]indol-5-ol, (1-methyl-(1,2,3,4-tetrahydroisoquinolinyl)carbamate

A degassed solution of eseroline (2.3 g) and 1,1'-carbonyldiimidazole (2.1 g) in 60 ml of dry dichloromethane was stirred at room temperature for one hour. This solution was treated with 1-methyl-1,2,3,4-tetrahydroisoquinoline (1.5 g) and stirred at 40°C for two hours, and thereafter the same amount of said isoquinoline derivative was added and the solution was stirred at reflux for three hours. The solution was concentrated and the residue purified by column chromatography over alumina to give 2.0 g of pale oil. This oil was crystallized from 50 ml of a 10:1 pentane/ether solution to give 1.6 g of white crystals, mp 105-108°C.

| ANALYSIS: | | | |
|---|---|---|---|
| Calculated for $C_{24}H_{29}N_3O_2$: | 73.62%C | 7.46%H | 10.73%N |
| Found: | 73.86%C | 7.48%H | 10.65%N |

**Claims**

1. A compound of the formula,

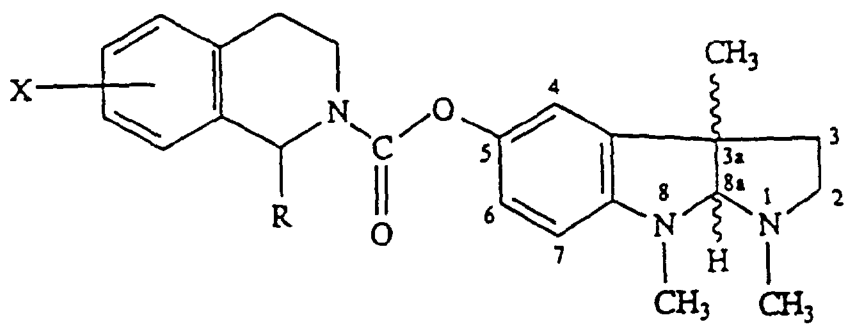

(I)

where R is hydrogen or a straight or branched alkyl group having 1 to 6 carbon atoms; and X is hydrogen, a straight or branched alkyl group having 1 to 6 carbon atoms, halogen, lower alkoxy or hydroxy, and wherein said compound of formula I is a cis derivative comprising either a 3*aS-cis* isomer, or a 3aR-*cis* isomer or mixtures of the 3aS-cis

and 3aR-cis isomers,
or a pharmaceutically acceptable acid addition salt thereof.

2. The compound as defined in Claim 1, where R is hydrogen or methyl.

3. The compound as defined in Claim 1, where X is hydrogen or halogen.

4. The compound as defined in Claim 1, where R is hydrogen or methyl, and X is hydrogen or halogen.

5. The compound as defined in Claim 1, which is (3aS-cis)-1,2,3,3a,8,8a-hexahydro-1,3a,8-trimethylpyrrolo[2,3-b] indol-5-ol,(1,2,3,4-tetrahydroisoquinolinyl)carbamate or the (3aR-cis) isomer thereof, or a mixture of the two isomers.

6. The compound as defined in Claim 1, which is (3aS-cis)-1,2,3,3a,8,8a-hexahydro-1,3a,8-trimethylpyrrolo[2,3-b] indol-5-ol, (1-methyl-1,2,3,4-tetrahydroisoquinolinyl)carbamate or the (3aR-cis) isomer thereof, or a mixture of the two isomers.

7. The compound as defined in Claim 1, which is (3aS-cis)-1,2,3,3a,8,8a-hexahydro-1,3a,8-trimethylpyrrolo[2,3-b] indol-5-ol, (1-ethyl-1,2,3,4-tetrahydroisoquinolinyl)carbamate or the (3aR-cis) isomer thereof, or a mixture of the two isomers.

8. The compound as defined in Claim 1, which is (3aS-cis)-1,2,3,3a,8,8a-hexahydro-1,3a,8-trimethylpyrrolo[2,3-b] indol-5-ol, (1-propyl-1,2,3,4-tetrahydroisoquinolinyl)carbamate or the (3aR-cis) isomer thereof, or a mixture of the two isomers.

9. The compound as defined in Claim 1, which is (3aS-cis)-1,2,3,3a,8,8a-hexahydro-1,3a,8-trimethylpyrrolo[2,3-b] indol-5-ol, (1-butyl-1,2,3,4-tetrahydroisoquinolinyl)carbamate or the (3aR-cis) isomer thereof, or a mixture of the two isomers.

10. The compound as defined in Claim 1, which is (3aS-cis)-1,2,3,3a,8,8a-hexahydro-1,3a,8-trimethylpyrrolo[2,3-b] indol-5-ol, (6-chloro-1,2,3,4-tetrahydroisoquinolinyl)carbamate or the (3aR-cis) isomer thereof, or a mixture of the two isomers.

11. The compound as defined in Claim 1, which is (3aS-cis)-1,2,3,3a,8,8a-hexahydro-1,3a,8-trimethylpyrrolo[2,3-b] indol-5-ol, (7-chloro-1,2,3,4-tetrahydroisoquinolinyl)carbamate or the (3aR-cis) isomer thereof, or a mixture of the two isomers.

12. The compound as defined in Claim 1, which is (3aS-cis)-1,2,3,3a,8,8a-hexahydro-1,3a,8-trimethylpyrrolo[2,3-b] indol-5-ol, (6-chloro-1-methyl-1,2,3,4-tetrahydroisoquinolinyl)carbamate or the (3aR-cis) isomer thereof, or a mixture of the two isomers.

13. The compound as defined in Claim 1, which is (3aS-cis)-1,2,3,3a,8,8a-hexahydro-1,3a,8-trimethylpyrrolo[2,3-b] indol-5-ol, (7-chloro-1-methyl-1,2,3,4-tetrahydroisoquinolinyl)carbamate or the (3aR-cis) isomer thereof, or a mixture of the two isomers.

14. The compound as defined in Claim 1, which is (3aS-cis)-1,2,3,3a,8,8a-hexahydro-1,3a,8-trimethylpyrrolo[2,3-b] indol-5-ol, (6-hydroxy-1,2,3,4-tetrahydroisoquinolinyl)carbamate or the (3aR-cis) isomer thereof, or a mixture of the two isomers.

15. The compound as defined in Claim 1, which is (3aS-cis)-1,2,3,3a,8,8a-hexahydro-1,3a,8-trimethylpyrrolo[2,3-b] indol-5-ol, (7-hydroxy-1,2,3,4-tetrahydroisoquinolinyl)carbamate or the (3aR-cis) isomer thereof, or a mixture of the two isomers.

16. The compound as defined in Claim 1, which is (3aS-cis)-1,2,3,3a,8,8a-hexahydro-1,3a,8-trimethylpyrrolo[2,3-b] indol-5-ol, (6-hydroxy-1-methyl-1,2,3,4-tetrahydroisoquinolinyl)carbamate or the (3aR-cis) isomer thereof, or a mixture of the two isomers.

17. The compound as defined in Claim 1, which is (3aS-cis)-1,2,3,3a,8,8a-hexahydro-1,3a,8-trimethylpyrrolo[2,3-b]indol-5-ol, (7-hydroxy-1-methyl-1,2,3,4-tetrahydroisoquinolinyl)carbamate or the (3aR-cis) isomer thereof, or a mixture of the two isomers.

**18.** A pharmaceutical composition comprising a compound as defined in any one of claims 1-17.

**19.** A compound as defined in any one of claims 1-17 for use in therapy.

**20.** The use of a compound as defined in any one of claims 1-17 for the preparation of a pharmaceutical composition for alleviating memory dysfunction characterized by a cholinergic deficit.

**21.** The use of a compound as defined in any one of claims 1-17 for the preparation of a pharmaceutical composition for the treatment of Alzheimer's disease.

**22.** The use of a compound as defined in any one of claims 1-17 for the preparation of a pharmaceutical composition for the treatment of glaucoma.

**23.** The use of a compound as defined in any one of claims 1-17 for the preparation of a pharmaceutical composition for the treatment of myasthenia gravis.

**24.** The use of a compound as defined in any one of claims 1-17 for the preparation of a pharmaceutical composition for the treatment of paralytic ileus.

**25.** A process for the preparation of a compound as defined in any one of claims 1-17 which comprises reacting sequentially a compound of the formula

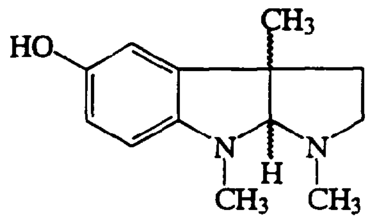

(Va)

with 1,1'-carbonyldiimidazole and thereafter with an amine of the formula

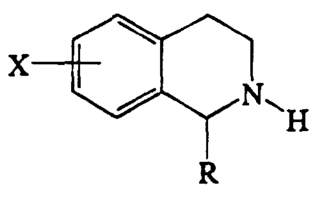

(VI)

wherein R and X are as defined in claim 1.

**26.** A process for the preparation of a pharmaceutical composition as defined in claims 18 and 20-24 comprising combining a compound as defined in any one of claims 1-17 with a pharmaceutically acceptable carrier.

### Patentansprüche

**1.** Verbindung der Formel

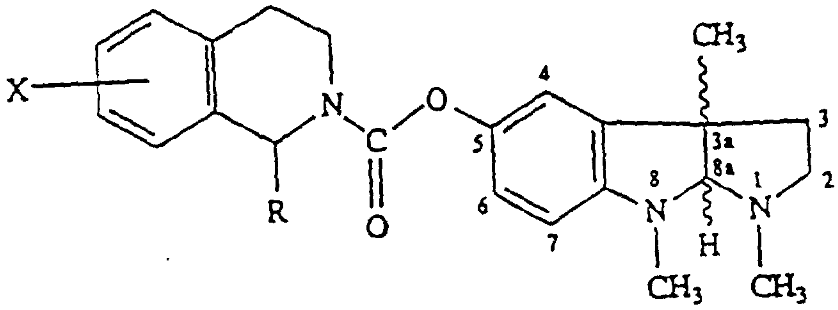

(I) ,

wobei R ein Wasserstoffatom oder ein gerader oder verzweigter Alkylrest mit 1 bis 6 Kohlenstoffatomen ist und X ein Wasserstoffatom, ein gerader oder verzweigter Alkylrest mit 1 bis 6 Kohlenstoffatomen, ein Halogenatom, ein Niederalkoxyrest oder eine Hydroxygruppe ist, wobei die Verbindung der Formel 1 ein cis-Derivat ist, das entweder ein 3aS-cis-Isomer oder ein 3aR-cis-Isomer oder Gemische des 3aS-cis-Isomers und des 3aR-cis-Isomers ist, oder ein pharmazeutisch verträgliches Säureadditionssalz davon.

**2.** Verbindung nach Anspruch 1, in der R ein Wasserstoffatom oder eine Methylgruppe ist.

**3.** Verbindung nach Anspruch 1, in der X ein Wasserstoff- oder Halogenatom ist.

**4.** Verbindung nach Anspruch 1, in der R ein Wasserstoffatom oder eine Methylgruppe und X ein Wasserstoff- oder Halogenatom ist.

**5.** Verbindung nach Anspruch 1, die (3aS-cis)-1,2,3,3a,8,8a-Hexahydro-1,3a,8-trimethylpyrrolo[2,3-b]indol-5-ol, (1,2,3,4-Tetrahydroisochinolinyl)carbamat oder das (3aR-cis)-Isomer davon ist, oder ein Gemisch der beiden Isomere.

**6.** Verbindung nach Anspruch 1, die (3aS-cis)-1,2,3,3a,8,8a-Hexahydro-1,3a,8-trimethylpyrrolo[2,3-b]indol-5-ol, (1-Methyl-1,2,3,4-tetrahydroisochinolinyl)carbamat oder das (3aR-cis)-Isomer davon ist, oder ein Gemisch der beiden Isomere.

**7.** Verbindung nach Anspruch 1, die (3aS-cis)-1,2,3,3a,8,8a-Hexahydro-1,3a,8-trimethylpyrrolo[2,3-b]indol-5-ol, (1-Ethyl-1,2,3,4-tetrahydroisochinolinyl)carbamat oder das (3aR-cis)-Isomer davon ist, oder ein Gemisch der beiden Isomere.

**8.** Verbindung nach Anspruch 1, die (3aS-cis)-1,2,3,3a,8,8a-Hexahydro-1,3a,8-trimethylpyrrolo[2,3-b]indol-5-ol, (1-Propyl-1,2,3,4-tetrahydroisochinolinyl)carbarnat oder das (3aR-cis)-Isomer davon ist, oder ein Gemisch der beiden Isomere.

**9.** Verbindung nach Anspruch 1, die (3aS-cis)-1,2,3,3a,8,8a-Hexahydro-1,3a,8-trimethylpyrrolo[2,3-b]indol-5-ol, (1-Butyl-1,2,3,4-tetrahydroisochinolinyl)carbamat oder das (3aR-cis)-Isomer davon ist, oder ein Gemisch der beiden Isomere.

**10.** Verbindung nach Anspruch 1, die (3aS-cis)-1,2,3,3a,8,8a-Hexahydro-1,3a,8-trimethylpyrrolo[2,3-b]indol-5-ol, (6-Chlor-1,2,3,4-tetrahydroisochinolinyl)carbamat oder das (3aR-cis)-Isomer davon ist, oder ein Gemisch der beiden Isomere.

**11.** Verbindung nach Anspruch 1, die (3aS-cis)-1,2,3,3a,8,8a-Hexahydro-1,3a,8-trimethylpyrrolo[2,3-b]indol-5-ol, (7-Chlor-1,2,3,4-tetrahydroisochinolinyl)carbamat oder das (3aR-cis)-Isomer davon ist, oder ein Gemisch der beiden Isomere.

**12.** Verbindung nach Anspruch 1, die (3aS-cis)-1,2,3,3a,8,8a-Hexahydro-1,3a,8-trimethylpyrrolo[2,3-b]indol-5-ol, (6-Chlor-1-methyl-1,2,3,4-tetrahydroisochinolinyl)carbamat oder das (3aR-cis)-Isomer davon ist, oder ein Gemisch der beiden Isomere.

**13.** Verbindung nach Anspruch 1, die (3aS-cis)-1,2,3,3a,8,8a-Hexahydro-1,3a,8-trimethylpyrrolo[2,3-b]indol-5-ol,

(7-Chlor-1-methyl-1,2,3,4-tetrahydroisochinolinyl)carbamat oder das (3aR-cis)-Isomer davon ist, oder ein Gemisch der beiden Isomere.

14. Verbindung nach Anspruch 1, die (3aS-cis)-1,2,3,3a,8,8a-Hexahydro-1,3a,8-trimethylpyrrolo[2,3-b]indol-5-ol, (6-Hydroxy-1,2,3,4-tetrahydroisochinolinyl)carbamat oder das (3aR-cis)-Isomer davon ist, oder ein Gemisch der beiden Isomere.

15. Verbindung nach Anspruch 1, die (3aS-cis)-1,2,3,3a,8,8a-Hexahydro-1,3a,8-trimethylpyrrolo[2,3-b]indol-5-ol, (7-Hydroxy-1,2,3,4-tetrahydroisochinohnyl)carbamat oder das (3aR-cis)-Isomer davon ist, oder ein Gemisch der beiden Isomere.

16. Verbindung nach Anspruch 1, die (3aS-cis)-1,2,3,3a,8,8a-Hexahydro-1,3a,8-trimethylpyrrolo[2,3-b]indol-5-ol, (6-Hydroxy 1-methyl-1,2,3,4-tetrahydroisochinolinyl)carbamat oder das (3aR-cis)-Isomer davon ist, oder ein Gemisch der beiden Isomere.

17. Verbindung nach Anspruch 1, die (3aS-cis)-1,2,3,3a,8,8a-Hexahydro-1,3a,8-trimethylpyrrolo[2,3-b]indol-5-ol, (7-Hydroxy-1-methyl-1,2,3,4-tetrahydroisochinolinyl)carbamat oder das (3aR-cis)-Isomer davon ist, oder ein Gemisch der beiden Isomere.

18. Arzneimittel, umfassend eine Verbindung nach einem der Ansprüche 1 bis 17.

19. Verbindung nach einem der Ansprüche 1 bis 17 zur Verwendung in der Therapie.

20. Verwendung einer Verbindung nach einem der Ansprüche 1 bis 17 zur Herstellung eines Arzneimittels zur Linderung von Gedächtnisfunktionsstörungen, die durch ein cholinerges Defizit gekennzeichnet sind.

21. Verwendung einer Verbindung nach einem der Ansprüche 1 bis 17 zur Herstellung eines Arzneimittels zur Behandlung von Alzheimer-Krankheit.

22. Verwendung einer Verbindung nach einem der Ansprüche 1 bis 17 zur Herstellung eines Arzneimittels zur Behandlung von Glaukom.

23. Verwendung einer Verbindung nach einem der Ansprüche 1 bis 17 zur Herstellung eines Arzneimittels zur Behandlung von Myasthenia gravis.

24. Verwendung einer Verbindung nach einem der Ansprüche 1 bis 17 zur Herstellung eines Arzneimittels zur Behandlung von paralytischer Ileus.

25. Verfahren zur Herstellung einer Verbindung nach einem der Ansprüche 1 bis 17, umfassend das aufeinanderfolgende Umsetzen einer Verbindung der Formel

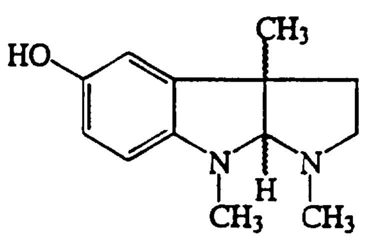

(Va)

mit 1,1'-Carbonyldiimidazol und danach mit einem Amin der Formel

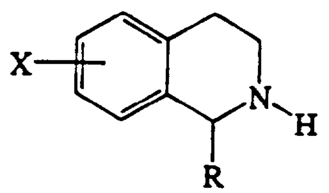

(VI)

,

wobei R und X die in Anspruch 1 definierte Bedeutung haben.

**26.** Verfahren zur Herstellung eines Arzneimittels nach den Ansprüchen 18 und 20 bis 24, umfassend die Kombination einer Verbindung nach einem der Ansprüche 1 bis 17 mit einem pharmazeutisch verträglichen Träger.

## Revendications

**1.** Composé de formule

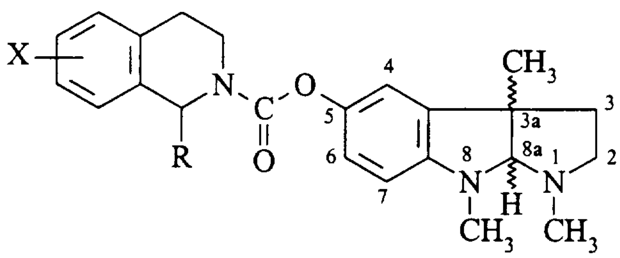

(I)

dans laquelle R est hydrogène ou un groupe alkyle droit ou ramifié ayant de 1 à 6 atomes de carbone ; et X est hydrogène, un groupe alkyle droit ou ramifié ayant de 1 à 6 atomes de carbone, halogène, alcoxy inférieur ou hydroxy et dans laquelle ledit composé de formule I est un dérivé cis comprenant soit l'isomère 3aS-cis, soit l'isomère 3aR-cis ou des mélanges des isomères 3aS-cis et 3aR-cis, ou un de ses sels d'addition avec des acides pharmaceutiquement acceptables.

**2.** Composé tel que défini dans la revendication 1, dans lequel R est hydrogène ou méthyle.

**3.** Composé tel que défini dans la revendication 1, dans lequel X est hydrogène ou halogène.

**4.** Composé tel que défini dans la revendication 1, dans lequel R est hydrogène ou méthyle et X est hydrogène ou halogène.

**5.** Composé tel que défini dans la revendication 1, qui est le (1,2,3,4-tétrahydroisoquinolinyl)carbamate de (3aS-cis)-1,2,3,3a,8,8a-hexahydro-1,3a,8-triméthylpyrrolo[2,3-b]indol-5-ol ou son isomère (3aR-cis) ou un mélange des deux isomères.

**6.** Composé tel que défini dans la revendication 1, qui est le (l-méthyl-1,2,3,4-tétrahydroisoquinolinyl)carbamate de (3aS-cis)-1,2,3,3a,8,8a-hexahydro-1,3a,8-triméthylpyrrolo[2,3-b]indol-5-ol ou son isomère (3aR-cis) ou un mélange des deux isomères.

**7.** Composé tel que défini dans la revendication 1, qui est le (1-éthyl-1,2,3,4-tétrahydroisoquinolinyl)carbamate de (3aS-cis)-1,2,3,3a,8,8a-hexahydro-1,3a,8-triméthylpyrrolo[2,3-b]indol-5-ol ou son isomère (3aR-cis) ou un mélange des deux isomères.

**8.** Composé tel que défini dans la revendication 1, qui est le (1-propyl-1,2,3,4-tétrahydroisoquinolinyl)carbamate de (3aS-cis)-1,2,3,3 a,8,8a-hexahydro-1,3a,8-triméthylpyrrolo[2,3-b]indol-5-ol ou son isomère (3aR-cis) ou un mélange des deux isomères.

**9.** Composé tel que défini dans la revendication 1, qui est le (1-butyl-1,2,3,4-tétrahydroisoquinolinyl)carbamate de (3aS-cis)-1,2,3,3a,8,8a-hexahydro-1,3a,8-triméthylpyrrolo[2,3-b]indol-5-ol ou son isomère (3aR-cis) ou un mélange des deux isomères.

**10.** Composé tel que défini dans la revendication 1, qui est le (6-chloro-1,2,3,4-tétrahydroisoquinolinyl)carbamate de (3aS-cis)-1,2,3,3a,8,8a-hexahydro-1,3a,8-triméthylpyrrolo[2,3-b]indol-5-ol ou son isomère (3aR-cis) ou un mélange des deux isomères.

**11.** Composé tel que défini dans la revendication 1, qui est le (7-chloro-1,2,3,4-tétrahydroisoquinolinyl)carbamate de (3aS-cis)-1,2,3,3a,8,8a-hexahydro-1,3a,8-triméthylpyrrolo[2,3-b]indol-5-ol ou son isomère (3aR-cis) ou un mélange des deux isomères.

**12.** Composé tel que défini dans la revendication 1, qui est le (6-chloro-1-méthyl-1,2,3,4-tétrahydroisoquinolinyl)carbamate de (3aS-cis)-1,2,3,3a,8,8a-hexahydro-1,3a,8-triméthylpyrrolo[2,3-b]indol-5-ol ou son isomère (3aR-cis) ou un mélange des deux isomères.

**13.** Composé tel que défini dans la revendication 1, qui est le (7-chloro-1-méthyl-1,2,3,4-tétrahydroisoquinolinyl)carbamate de (3aS-cis)-1,2,3,3 a,8,8a-hexahydro-1,3a,8-triméthylpyrrolo[2,3-b]indol-5-ol ou son isomère (3aR-cis) ou un mélange des deux isomères.

**14.** Composé tel que défini dans la revendication 1, qui est le (6-hydroxy-1,2,3,4-tétrahydroisoquinolinyl)carbamate de (3aS-cis)-1,2,3,3a,8,8a-hexahydro-1,3a,8-triméthylpyrrolo[2,3-b]indol-5-ol ou son isomère (3aR-cis) ou un mélange des deux isomères.

**15.** Composé tel que défini dans la revendication 1, qui est le (7-hydroxy-1,2,3,4-tétrahydroisoquinolinyl)carbamate de (3aS-cis)-1,2,3,3a,8,8a-hexahydro-1,3a,8-triméthylpyrrolo[2,3-b]indol-5-ol ou son isomère (3aR-cis) ou un mélange des deux isomères.

**16.** Composé tel que défini dans la revendication 1, qui est le (6-hydroxy-1-méthyl-1,2,3,4-tétrahydroisoquinolinyl) carbamate de (3aS-cis)-1,2,3,3a,8,8a-hexahydro-1,3a,8-triméthylpyrrolo[2,3-b]indol-5-ol ou son isomère (3aR-cis) ou un mélange des deux isomères.

**17.** Composé tel que défini dans la revendication 1, qui est le (7-hydroxy-1-méthyl-1,2,3,4-tétrahydroisoquinolinyl) carbamate de (3aS-cis)-1,2,3,3a,8,8a-hexahydro-1,3a,8-triméthylpyrrolo[2,3-b]indol-5-ol ou son isomère (3aR-cis) ou un mélange des deux isomères.

**18.** Composition pharmaceutique comprenant un composé tel que défini dans l'une quelconque des revendications 1 à 17.

**19.** Composé tel que défini dans l'une quelconque des revendications 1 à 17 pour une utilisation en thérapie.

**20.** Utilisation d'un composé tel que défini dans l'une quelconque des revendications 1 à 17 pour la préparation d'une composition pharmaceutique pour atténuer les dysfonctionnements de la mémoire caractérisés par un déficit cholinergique.

**21.** Utilisation d'un composé tel que défini dans l'une quelconque des revendications 1 à 17 pour la préparation d'une composition pharmaceutique pour le traitement de la maladie d'Alzheimer.

**22.** Utilisation d'un composé tel que défini dans l'une quelconque des revendications 1 à 17 pour la préparation d'une composition pharmaceutique pour le traitement du glaucome.

**23.** Utilisation d'un composé tel que défini dans l'une quelconque des revendications 1 à 17 pour la préparation d'une composition pharmaceutique pour le traitement de la myasthénie grave.

**24.** Utilisation d'un composé tel que défini dans l'une quelconque des revendications 1 à 17 pour la préparation d'une composition pharmaceutique pour le traitement de l'iléus paralytique.

**25.** Procédé pour la préparation d'un composé tel que défini dans l'une quelconque des revendication 1 à 17, qui

comprend la réaction séquentielle d'un composé de formule

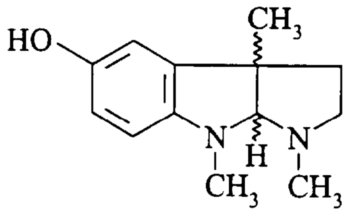

(Va)

avec le 1,1'-carbonyldiimidazole et ensuite avec une amine de formule

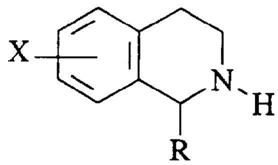

(VI)

dans laquelle R et X sont tels que définis dans la revendication 1.

**26.** Procédé pour la préparation d'une composition pharmaceutique telle que définie dans les revendications 18 et 20 à 24, comprenant la combinaison d'un composé tel que défini dans l'une quelconque des revendications 1 à 17 avec un excipient pharmaceutiquement acceptable.